# EUROPÄISCHE PATENTSCHRIFT

(11) **EP 0 724 585 B1**
(45) Veröffentlichungstag und Bekanntmachung des Hinweises auf die Patenterteilung: **21.05.1997**
(21) Anmeldenummer: 94924837.1
(22) Anmeldetag: 03.08.1994
(51) Int. Cl.: C07D 487/04, C11D 3/39, C07D 471/04, C07D 519/00

(54) **HYDANTOINDERIVATE UND DEREN VERWENDUNG ALS BLEICHAKTIVATOR**
HYDANTOIN DERIVATIVES AND THEIR USE AS BLEACH ACTIVATORS
DERIVES DE L'HYDANTOINE ET APPLICATION COMME ACTIVATEURS DU BLANCHIMENT

(30) Priorität: 15.11.1993 DE 4338920
(43) Veröffentlichungstag der Anmeldung: 07.08.1996
(73) Patentinhaber: Degussa Aktiengesellschaft, 60311 Frankfurt (DE)
(72) Erfinder: DEL GROSSO, Michael, D-63579 Freigericht (DE); TANNER, Herbert, D-63457 Hanau (DE); KOTTENHAHN, Matthias, D-63579 Freigericht (DE); THIELE, Georg, D-63452 Hanau (DE)
(86) Internationale Anmeldenummer: EP9402568
(87) Internationale Veröffentlichungsnummer: WO9514021

(56) Entgegenhaltungen:
- DE-A- 1 802 576

## Beschreibung

Die Erfindung richtet sich auf neue Hydantoinderivate auf der Basis N-acylierter Hydantoine, welche als Aktivatoren für anorganische Persauerstoffverbindungen, wie insbesondere Wasserstoffperoxid und Wasserstoffperoxid freisetzende Verbindungen, verwendet werden können. Die Erfindung richtet sich ferner auf diese Aktivatoren enthaltende Bleich-, Wasch-, Reinigungs- und Desinfektionsmittel.

Anorganische Persauerstoffverbindungen werden als Oxidationsmittel in Bleich-, Wasch-, Reinigungs- und Desinfektionsmitteln eingesetzt, um die Wirkung derartiger Mittel zu verbessern. Als Persauerstoffverbindungen kommen insbesondere Wasserstoffperoxid und solche Stoffe zum Einsatz, welche in wäßriger Lösung Wasserstoffperoxid freisetzen, wie Perborate und Percarbonate. Die Wirkung der anorganischen Persauerstoffverbindungen hängt außer vom pH-Wert maßgeblich von der Temperatur ab. Während bei Temperaturen oberhalb etwa 80 °C eine gute Wirkung erzielt wird, ist bei der Verwendung der genannten anorganischen Persauerstoffverbindung bei niedrigeren Temperaturen, insbesondere bei 60 °C oder 40 °C oder darunter, die Mitverwendung sogenannter Aktivatoren erforderlich. Bei den Aktivatoren handelt es sich überwiedend um N-Acyl- oder O-Acylverbindungen. In wäßriger Phase bilden sich aus den anorganischen Persauerstoffverbindungen und den Aktivatoren Percarbonsäuren, welche ein höheres Oxidationspotential als H₂O₂ und dieses freisetzende Verbindungen aufweisen und daher auch im Niedertemperaturbereich eine gute Wasch-, Reinigungs-, Bleich- und Desinfektionswirkung entfalten.

Unter den N-Acylverbindungen sind zahlreiche Stoffklassen als Aktivatoren vorgeschlagen worden, darunter: N,N,N',N'-Tetraacetyl-ethylendiamin (TAED), N,N,N',N'-Tetraacetylglykoluril (TAGU), N,N'-Di(alkoxycarbonyl)-hydantoine (US 3,928,223), N-Mono- und N,N'-Di(C₁- bis C₄)alkanoylhydantoine (DE-A 14 67 582 und DE-C 19 49 561), N-(C₁- bis C₄-)Alkyloxycarbonylsuccinimide (US 3,928,223) und N,N'-Diacyl-2,5-diketopiperazine (DE-A 20 38 106). Trotz dieser Vielfalt konnte sich im Markt von den N-Acylverbindungen im wesentlichen nur TAED durchsetzen.

Sowohl beim TAED als auch bei etlichen anderen N-Acylverbindungen werden nicht alle Acylgruppen in die entsprechenden Percarbonsäuren überführt - aus TAED entstehen maximal 2 Mol Peressigsäure. Bei den vorbekannten Diacyl-hydantoinen, wie dem N,N'-Diacetyl-hydantoin, wurde festgestellt, daß diese im Aktivatortest bei 30 °C weniger als 1 Mol Percarbonsäure freizusetzen vermögen - etwa 0,9 Mol Peressigsäure im Fall des N,N'-Diacetyl-hydantoins.

In Anbetracht des wachsenden Bedarfs an Wasch-, Bleich-, Reinigungs- und Desinfektionsmitteln für den Niedrigtemperaturbereich besteht auch ein Interesse an weiteren Aktivatoren auf der Basis von N-Acylverbindungen, welche im wesentlichen an das Eigenschaftbild des TAED heranreichen und/oder dieses in dem einen oder anderen Punkt übertreffen. Da bekannt ist, daß die Struktur der Persäure einen gewissen Einfluß auf das Oxidations- und Bleichvermögen gegenüber beispielsweise unterschiedlichen Anschmutzungen hat, besteht zusätzlich ein Interesse an solchen Aktivatoren, welche in Gegenwart anorganischer Persauerstoffverbindungen zwei oder mehr unterschiedliche Persäuren in situ freizusetzen in der Lage sind, um im Vergleich zu üblichen, nur eine Art Persäure freisetzenden Aktivatoren zu einer synergistischen Wirkung zu gelangen, ohne zwei unterschiedliche Aktivatoren einsetzen zu müssen. Die bereitzustellenden Aktivatoren sollen nach Möglichkeit aus leicht verfügbaren und biologisch abbaubaren Rohstoffen zugänglich sein.

Gefunden wurden Hydantoinderivate der allgemeinen Formel (I) 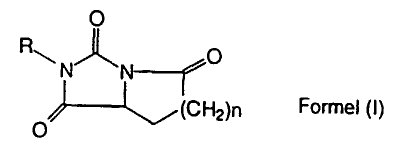 worin bedeuten
- n: die Zahl 1 oder 2 und
- R: Wasserstoff, (C₁- bis C₄)Alkyloxycarbonyl oder eine Acylgruppe der allgemeinen Formel R'-CO-, wobei
- R': für einen linearen oder verzweigten gesättigten oder ungesättigten Kohlenwasserstoffrest mit 1 bis 11 C-Atomen, dessen längste Kette durch eine oder mehrere Etherbrücken unterbrochen sein kann und der zusätzlich ein oder zwei Substituenten aus der Reihe Carboxy, C₁- bis C₃-Alkoxy, -N((C₁-C₃)alkyl)₃⁺Cl⁻, Phenyl oder mit ein oder zwei Carboxy- oder Sulfogruppen subtituiertes Phenyl aufweisen kann,
oder
für Phenyl oder ein- oder zweifach substituiertes Phenyl, wobei die Substituenten C₁- bis C₄-Alkyl, Carboxy, -SO₃H, -NO_{2,} -N((C₁-C₃)alkyl)₃⁺Cl⁻ oder -CN sein können,
oder
für einen Rest der Formel (II) 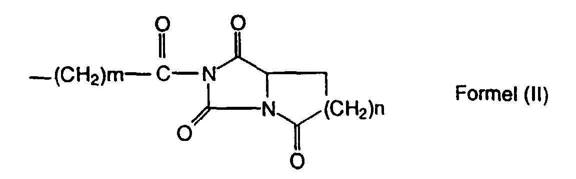 in welcher m eine ganze Zahl zwischen 2 und 10 und n die Zahl 1 oder 2 bedeuten, steht.

Die erfindungsgemäßen Hydantoinderivate waren bisher nicht bekannt. Überraschenderweise zeichnen sie sich durch eine wesentlich höhere Aktivatorleistung aus als vorbekannte und als Aktivatoren bekannte N,N'-Dialkanoyl-hydantoine, wie N,N'-Diacetyl-hydantoin. Erfindungsgemäße Aktivatoren auf der Basis der N-acylierten bicyclischen Hydantoinderivate bilden unter Testbedingungen bei 30 °C in Gegenwart von Natriumperborat-monohydrat (Pbmh) mehr als ein Äquivalent Persäure. Beispielsweise werden aus 2-Acetyl-dihydropyrrolo[1,2-c]imidazol-1,3,5-trion etwa 1,8 Äquivalente Persäure freigesetzt, wobei ein Teil auf Peressigsäure entfällt und ein Teil auf 3-(5-Hydantoinyl)perpropionsäure. Diese Persäurefreisetzungsrate war in Kenntnis der unbefriedigenden Persäurebildung aus vorbekannten N,N'-Dialkanoyl-hydantoinen nicht zu erwarten.

Auch im für die Praxis bedeutsamen Waschtest von Geweben mit Testanschmutzungen wurde die überlegene Wirkung erfindungsgemäßer N-acylierter bicyclischer Hydantoinderivate bestätigt. Im Mittel von sechs unterschiedlichen Testanschmutzungen wird von einigen erfindungsgemäßen Aktivatoren, insbesondere jenen der Formel (I) mit n gleich 1 und R gleich Acetyl, Propionyl, Butyryl, Phenyl, o- oder p-Carboxyphenyl das Leistungsniveau des als Standart dienenden TAED nicht nur erreicht, sondern zum Teil deutlich überschritten.

Einige Verbindungen auf der Basis des Dihydropyrrolo[1,2-c]imidazol-Ringsystems sind bekannt - gemäß US 5,110,823 Dihydropyrrolo[1,2-c]imidazol-3,5(1H,6H)-dion und dessen 1-Alkoxycarbonylderivate - jedoch ist das erfindungsgemäße Ringsystem durch eine weitere Carbonylgruppe im Imidazolring gekennzeichnet, welche für die Aktivatorwirkung der N-acylierten Verbindungen wichtig ist. Hinweise auf eine durch Perhydrolyse mögliche Ringöffnung und die Verwendbarkeit solcher Stoffe als Aktivatoren lassen sich diesem Dokument nicht entnehmen.

Die erfindungsgemäßen Hydantoinderivate basieren auf Dihydro-pyrrolo[1,2-c]imidazol-1,3,5-trion (1) bzw. dessen 2-N-acylierten Derivaten, wenn n in der allgemeinen Formel (I) gleich 1 ist oder auf Tetrahydro-pyridino [1,2-c]imidazol-1,3,5-trion (8), wenn n gleich 2 ist.

Bevorzugte Verbindungen sind solche mit n gleich 1, weil diese aus leicht zugänglichen Rohstoffen, nämlich Glutaminsäure oder Na-glutamat (fermentativ herstellbar), erhältlich sind, wohingegen sich Verbindungen mit n gleich 2 aus der 2-Aminoadipinsäure, die in an sich bekannter Weise synthetisch hergestellt werden kann, gewinnen lassen.

Erfindungsgemäße Hydantoinderivate mit n gleich 1, welche in Form der möglichen Stereoisomeren oder Gemischen derselben vorliegen können, lassen sich aus fermentativ hergestellter L-Glutaminsäure oder Na-L-Glutamat oder aus synthetisch hergestellter D,L-Glutaminsäure oder dessen Salz herstellen. Nach Überführung der Säure in das Na-Salz, etwa durch Zugabe von Natronlauge in wäßriger Phase, schließt sich eine konventionelle Hydantoinsynthese an, vorzugsweise unter Verwendung eines Alkalicyanats: In wäßriger Phase wird Na-Glu mit MeOCN (Me = Li, Na, K) bei erhöhter Temperatur umgesetzt, anschließend das Reaktionsgemisch mit HCl behandelt, wobei die 3(Hydantoin-5-yl)-propionsäure (HPS) auskristallisiert. Zur Herstellung von (1) wird das vorgenannte Produkt (HPS) in Eisessig mit Essigsäureanhydrid umgesetzt. Die acylierten Verbindungen von (1), also Stoffe mit R = Acyl oder Alkoxycarbonyl, sind in für den Fachmann bekannter Weise durch Umsetzung von (1) mit einem Säurehalogenid oder -anhydrid zu gewinnen. Zur Acylierung von (1) wird (1) zweckmäßigerweise zunächst am Imidstickstoff deprotoniert - etwa mittels eines Alkalihydrids und dann in einem dipolaren Lösungsmittel mit dem Säurehalogenid umgesetzt.

Erfindungsgemäße Hydantoinderivate der Formel (I) mit n gleich 2 lassen sich in prinzipiell analoger Weise, wie oben beschrieben, herstellen. 2-Aminoadipinsäure wird mittels eines Alkalicyanats in 4-(Hydantoin-5-yl)-buttersäure überführt; die Cyclisierung zum Tetrahydropyridinoring erfolgt wieder mittels wasserabspaltender Mittel, wie Essigsäureanhydrid. Die N-Acylierung des Imidstickstoffs erfolgt in bekannter Weise.

Bezüglich ihrer Aktivierung werden die 2-N-acylierten Verbindungen von (1) beziehungsweise (8) bevorzugt. Die Bedeutung des Rests R' in der Acylgruppe R'-CO- kann sehr vielseitig sein und einen aliphatischen, olefinischen, cycloaliphatischen oder aromatischen Rest umfassen, wobei die genannten Reste selbst ein oder zwei Substituenten aus der bereits genannten Reihe aufweisen können. Besonders zweckmäßig steht R' für eine lineare Alkylgruppe mit 1 bis 11 C-Atomen, wie Methyl, Ethyl, Propyl, Butyl, Pentyl, Hexyl, Heptyl, Octyl, Decyl, Undecyl. Olefinische Kohlenwasserstoffreste, wie Vinyl, Allyl, Propen-2-yl, sind möglich, werden aber weniger bevorzugt. Die Zahl und Größe der Substituenten am Kohlenwasserstoffrest wird im allgemeinen niedrig gehalten, um das Molekulargewicht der resultierenden Percarbonsäure zu begrenzen. Als aromatischer Rest R' wird außer Phenyl substituiertes Phenyl mit vorzugsweise einer oder zwei Carboxy- oder Sulfogruppen bevorzugt.

Besonders bevorzugte Verbindungen sind die von (1) abgeleiteten N-Acylhydantoine mit einer C₂- bis C₉-n-Alkanoyl- oder einer Benzoyl- oder o- oder p-Carboxybenzoylgruppe; unter diese Gruppe fallen die Aktivatoren (2) bis (5) gemäß den Beispielen 2 bis 5.

Verbindungen vom Formeltyp (II) sind besonders zweckmäßig, wenn n für 2, 3, 4 und 10 steht. Die zur Herstellung dieser Verbindungen erforderlichen Dicarbonsäuren, nämlich Bernsteinsäure, Glutarsäure, Adipinsäure und Dodecandisäure, deren Dichloride mit 2 Mol Dihydropyrrolo[1,2-c]imidazol-1,3,5-trion unter Bildung von (II) umgesetzt werden, sind leicht verfügbar. Beispielsweise läßt sich N,N'-Dodecan-1,12-dioyl-di-2-(dihydropyrrolo[1,2-c]imidazol-1,3,5-trion, aus welchem in Gegenwart von Wasserstoffperoxid die als besonders bleichwirksam bekannte Diperoxydodecandisäure in situ gebildet wird, aus 3-(Hydantoin-5-yl)-propionsäure und dem α,ω-Dodecandicarbonsäuredichlorid herstellen.

Die erfindungsgemäße Verwendung betrifft die Aktivierung anorganischer Persauerstoffverbindungen, insbesondere H₂O₂ und solche Verbindungen, welche in wäßriger Phase Wasserstoffperoxid freisetzen. Hervorzuheben sind Perborate, insbesondere Natriumperborat-monohydrat, Natriumperborat-tetrahydrat, superoxidiertes Natriumperborat, und Natriumpercarbonat (2 Na₂CO₃·3 H₂O₂). Einsetzbar sind auch Perphosphate, Persilikate und Persulfate. Bei der Aktivierung können auch mehrere anorganische Perverbindungen zugegen sein.

Bei der Verwendung werden anorganische Persauerstoffverbindungen und Aktivatoren in einem Verhältnis von 1 Mol Aktivsauerstoff zu 0,05 bis 1 Mol, vorzugsweise 1 zu 0,1 bis 0,5 Mol Aktivator eingesetzt.

Die erfindungsgemäß zu verwendenden Aktivatoren können zur Aktivierung in reiner Form oder mit Hilfsstoffen, wie Granulierhilfsmitteln, Stabilisatoren, pH-regulierenden Stoffen, eingesetzt werden; geeignete Zugabeformen sind Pulver, Pasten, Tabletten, Granulate oder umhüllte Granulate.

Je nach Verwendungszweck können die Aktivatoren und anorganischen Persauerstoffverbindungen außer in rein wäßriger Phase auch in wäßrig-organischer Phase zum Einsatz kommen. Ein rein wäßriges Milieu liegt bei den üblichen Wasch-, Bleich- und Reinigungsflotten vor. Ein wäßrigorganisches Milieu kann bei Anwendungen zur Desinfektion sowie bei technischen Oxidationsprozessen zweckmäßig sein.

Der pH-Wert des Reaktionsmediums kann zwischen etwa 4 und 13 liegen, vorzugsweise wird aber im alkalischen Bereich, meist bei pH 8 bis 11, gearbeitet, da in diesem Bereich sowohl die in situ-Bildung der organischen Persäure gut abläuft als auch die Stabilität der Perverbindungen befriedigend ist.

Ein weiterer Gegenstand der Erfindung richtet sich auf Bleich-, Wasch-, Reinigungs- und Desinfektionsmittel, welche eine anorganische Persauerstoffverbindung und einen Aktivator aus der Reihe der erfindungsgemäßen Hydantoinderivate der Formel (I) oder (II) enthalten.

In den Mitteln können ein oder mehrere anorganische Persauerstoffverbindungen sowie ein oder mehrere Aktivatoren, darunter mindestens ein erfindungsgemäßer und bei Bedarf auch handelsübliche oder andere vorbekannte Aktivatoren enthalten sein.

Erfindungsgemäße Aktivatoren und anorganische Persauerstoffverbindungen können mit allen üblichen Bestandteilen von Wasch- und Bleichmitteln kombiniert werden, um zu Wasch- und Bleichmitteln zu gelangen, welche zur Textilbehandlung im Nieder- und Mitteltemperaturbereich, aber auch zur Kochwäsche geeignet sind.

Hauptbestandteile solcher Wasch- und Bleichmittel sind, neben den erwähnten Perverbindungen und Aktivatoren, Gerüststubstanzen (Builder) und Tenside. Unter den Buildern sind insbesondere Natriumaluminiumsilikate (Zeolithe), kondensierte Phosphate, Alkalisilikate, Alkalicarbonate, komplexierende Aminocarbonsäuren, Polyphosphonsäuren, mehrwertige Hydroxycarbonsäuren sowie Polycarbonsäuren und Salze der genannten Säuren zu nennen. Unter den Tensiden sind insbesondere nichtionische Tenside, wie Fettalkohol- und Alkylphenol-polyethylenglykolether sowie langkettige Alkylglykoside, und anionische Tenside, wie Alkylbenzolsulfonate und Sulfate von Fettalkoholen und Polyethylenglykolmonoethern, hervorzuheben. Weitere Stoffe der Wasch- und Bleichmittel sind Elektrolyte, pH-regulierende Stoffe, Stabilisatoren, Schaumregulatoren, Vergrauungsinhibitoren, optische Aufheller, Enzyme, Avivagemittel. Die in derartigen Mitteln zu verwendenden Stoffe und Einsatzmengen sind dem Fachmann bekannt - eine Übersicht nebst Literatur vermittelt H.G. Hauthal in "Chemie in unserer Zeit" 26 (1992) Nr.6, 293-303).

Üblicherweise setzen sich erfindungsgemäße Wasch- und Bleichmittel etwa wie folgt zusammen:
- 5 bis 30 Gew.-%,: vorzugsweise 10 bis 25 Gew.-% anionische und/oder nichtionische Tenside,
- 5 bis 60 Gew.-%,: vorzugsweise 20 bis 40 Gew.-% Gerüstsubstanzen aus der Gruppe Natriumaluminiumsilikate, kondensierte Phosphate, Alkalisilikate, Alkalicarbonate, und deren Mischungen,
- 0 bis 20 Gew.-%,: vorzugsweise 1 bis 8 Gew.-% Gerüstsubstanzen aus der Gruppe komplexierender Aminocarbonsäuren, Polyphosphonsäuren, Polycarbonsäuren oder deren Salze sowie deren Mischungen,
- 2 bis 35 Gew.-%,: vorzugsweise 10 bis 25 Gew.-% anorganische Persauerstoffverbindungen aus der Gruppe Natriumperborate und Natriumpercarbonat,
- 0,3 bis 20 Gew.-%,: vorzugsweise 1 bis 10 Gew.-% erfindungsgemäße Hydantoinderivate, vorzugsweise N-acylierte Hydantoinderivate der Formel (I) mit n gleich 1 als Aktivatoren
- ad 100 %: übliche Hilfsstoffe und Wasser

Reine Bleichmittel, wie sie als Zusatzmittel für bleichmittelfreie Waschmittel zur Anwendung gelangen können, setzen sich im allgemeinen wie folgt zusammen:
- 5 bis 50 Gew.-%,: insbesondere 15 bis 35 Gew.-%, anorganische Persauerstoffverbindungen, insbesondere Na-perborat-monohydrat oder - tetrahydrat oder/und Natriumpercarbonat,
- 2 bis 50 Gew.-%,: insbesondere 5 bis 25 Gew.-% erfindungsgemäße Hydantoinderivate, vorzugsweise N-acylierte Hydantoinderivate der Formel (I) mit n gleich 1 als Aktivatoren,
- 0 bis 5 Gew.-%: Peroxidstabilisatoren, wie Wasserglas und Komplexbildner,
- 0 bis 40 Gew.-%: pH-regulierende Mittel
- ad 100 Gew.-%: andere übliche Hilfsstoffe.

Erfindungsgemäße Reinigungsmittel enthalten im allgemeinen Tenside, Builder, Persauerstoffverbindungen und erfindungsgemäße Aktivatoren; Scheuermittel enthalten zusätzlich abrasiv wirkende Bestandteile.

Erfindungsgemäße Desinfektionsmittel basieren im allgemeinen auf einer Kombination aus anorganischen Perverbindungen und erfindungsgemäße Aktivatoren sowie Hilfsstoffen aus der Reihe der Stabilisatoren, Tenside, pH-regulierenden Stoffe und, sofern erwünscht, organischen Lösungsmitteln und anderen mikrobioziden Stoffen als die aus den Aktivatoren und Perverbindungen entstehenden Persäuren.

### Beispiel 1

### Dihydro-pyrrolo[1,2-c]imidazol-1,3,5-trion (Formel (I), R = H) (1)

Ein Gemisch aus 130 g (0,76 Mol) 3-(Hydantoin-5-yl)propionsäure, 79 g (0,74 Mol) Essigsäureanhydrid und 250 g Eisessig wurden 24 h unter Rückfluß erhitzt und anschließend die Reaktionsmischung auf Raumtemperatur abgekühlt. Der ausgefallene Niederschlag wurde abfiltriert und getrocknet. Man erhielt 76 g (66 % d. Th.) als farblosen Feststoff.
Die 1H-NMR-spektroskopischen Daten stehen mit der Struktur im Einklang.

### Beispiel 2

### 2-Acetyl-dihydro-pyrrolo[1,2-c]imidazol-1,3,5-trion (Formel (I) R = Acetyl) (2)

17 g (0,1 Mol) 3-(Hydantoin-5-yl)propionsäure und 60 g (0,6 Mol) Essigsäureanhydrid wurden 3 h unter Rückfluß erhitzt. Durch Destillation unter Normalbedingungen entfernte man 40 ml des Lösungsmittels und ließ die Reaktionslösung bei Raumtemperatur stehen. Der ausgefallene Niederschlag wurde abgesaugt, mit Methanol gewaschen und getrocknet. Man erhielt 13,8 g (71 % d. Th.) als farblos kristallinen Feststoff.
Die ¹H-NMR-spektroskopischen Daten stehen mit der Struktur im Einklang.

### Beispiel 3

### 2-Nonanoyl-dihydro-pyrrolo[1,2-c]imidazol-1,3,5-trion (Formel (I), R = Nonanoyl) (3)

4 g (26 mMol) Dihydro-pyrrolo[1,2-c]imidazol-1,3,5-trion (1) wurden in 30 ml Dimethylformamid gelöst, bei Eisbadtemperatur portionsweise mit 0,69 g (29 mMol) Natriumhydrid versetzt und 1 h bei Raumtemperatur gerührt. Anschließend tropfte man 4,8 g (27 mMol) Nonanoylchlorid in 10 ml Dimethylformamid zu und ließ 2 h bei Raumtemperatur und 1 h bei 80 °C rühren. Danach entfernte man weitgehend das Lösungsmittel im Rotationsverdampfer (70 °C, 10 mbar) und nahm den Rückstand in Methylenchlorid auf. Die Suspension wurde mit Wasser gewaschen, die organische Phase getrocknet (MgSO₄) und nach Entfernen des Lösungsmittels im Rotationsverdampfer der Rückstand aus Ethanol umkristallisiert. Man erhielt 3 g als Feststoff.
Die ¹H-NMR-spektroskopischen Daten stehen mit der Struktur im Einklang.

### Beispiel 4

### 2-Propionyl-dihydro-pyrrolo[1,2-c]imidazol-1,3,5-trion (4)

Analog Beispiel 3 wurde (1) (aus Beispiel 1) zunächst innerhalb 2 h in DMF bei 20 °C mit NaH deprotoniert und anschließend bei 20 °C mit Propionylchlorid umgesetzt; Nachreaktion 2 h bei 80 °C; Aufarbeitung wie Beispiel 3. Man erhält (4) (gemäß ¹H-NMR-Spektrum) als kristallinen Feststoff.

### Beispiel 5

### 2-Benzoyl-dihydro-pyrrolo[1,2-c]imidazol-1,3,5-trion (5)

Analog Beispiel 4 wurde (1) mit NaH und Benzoylchlorid umgesetzt, woraus der feste Stoff (5) resultierte.

### Beispiel 6

### 2-Ethoxycarbonyl-dihydro[1,2-c]imidazol-1,3,5-trion (6)

Analog Beispiel 4 wurde (1) mit NaH und Chlorameisensäureethylester umgesetzt, woraus gemäß ¹H-NMR-Spektrum (6) in Form eines viskosen Öls erhalten wurde.

### Beispiel 7

### 3-(Hydantoin-5-yl)-propionsäure (7)

L-Glutaminsäure (Glu), gewonnen durch Fermentation, wurde mittels Natronlauge in Wasser in Natrium-L-glutamat überführt. Anschließend wurde in Wasser mit Natriumcyanat (20 Mol-% Überschuß) 2 h bei 80 bis 85 °C behandelt. Das Reaktionsgemisch wurde mit konz. HCl auf pH 0 gestellt und 2 h unter Rückfluß gekocht. Nach dem Abkühlen wird kristallines (7) in üblicher Weise isoliert.

### Beispiel 8

### Persäurebildung aus den Aktivatoren (1) bis (6) gemäß Beispielen 1 bis 6 und 1,3-Diacetylhydantoin (Vergleichsbeispiel VB)

Zu jeweils 1 Liter Wasser, auf 30 °C temperiert, wurden 8 g bleichmittelfreies zeolithhaltiges Waschmittel-Turmpulver, 1,5 g Natriumperborat-monohydrat (Pbmh) und 0,5 g Aktivator zugegeben. In bestimmten Zeitabständen wurden 100 ml-Proben entnommen, diese sofort auf ein Gemisch aus 250 g Eis und 15 ml Eisessig gegeben und anschließend nach Zugabe von Kaliumjodid mit 0,1 n Natriumthiosulfatlösung und Stärke als Indikator titriert. Unter den angegebenen Bedingungen wurden nur die in situ gebildeten Persäuren erfaßt. Die Ergebnisse - Gehalt Äquivalente Persäure in Zeitabhängigkeit - folgen aus Tabelle 1.

**Tabelle 1:**

| Persäurebildung und -stabilität bei 30 °C | | | | | | | |
|---|---|---|---|---|---|---|---|
| Aktivator gemäß Beispiel | (1) | (2) | (3) | (4) | (5) | (6) | (VB) |
| Zeit (Min) | Persäure - Äquivalente (gemessen) | | | | | | |
| 2 | 0,50 | 1,45 | 0,22 | 1,31 | 0,58 | 0,12 | 0,78 |
| 10 | 0,90 | 1,85 | 0,54 | 1,70 | 1,21 | 0,41 | 0,91 |
| 20 | 0,92 | 1,82 | 0,81 | 1,72 | 1,45 | 0,72 | 0,90 |
| 30 | 0,89 | 1,75 | 0,90 | 1,63 | 1,54 | 0,94 | 0,88 |
| 40 | 0,86 | 1,65 | 1,00 | 1,59 | 1,54 | 1,05 | |
| 50 | | | 1,05 | | 1,52 | 1,10 | |

Aus dem Gehalt an Persäure in Zeitabhängigkeit folgt, daß der vorbekannte Aktivator Diacetylhydantoin (VB) anstelle der theoretisch möglichen 2 Äquivalente nur etwa 0,9 Äquivalente Persäure bildet, wogegen bevorzugte erfindungsgemäße Aktivatoren mit R = Acyl bis zu 1,85 Äquivalente Persäure gebildet werden.

### Beispiel 9

Bleichleistung der Aktivatoren
Dihydropyrrolo[1,2-c]imidazol-1,3,5-trion (1) 2-Acetyldihydro-pyrrolo[1,2-c]imidazol-1,3,5-trion (2) und zum Vergleich Diacetylhydantoin (VB) sowie TAED bei 40 °C.

Untersucht wurde die Bleichwirkung an sechs
Testanschmutzungen auf Baumwolle (Bandy-black-Lehm; WFK-BW: Tee, Curry, Rotwein, Kaffee, Tomatenketchup) im Launderometer (Atlas).
Temperatur: 40 °C
Zugabemengen: Waschmittelbestandteile in g/l Flotte

| | |
|---|---|
| Alkylbenzolsulfonate | 0,52 |
| Fettalkoholethoxylate | 0,35 |
| Seife | 0,10 |
| Zeolith A | 1,52 |
| Polycarboxylate | 0,18 |
| Soda | 0,76 |
| Na- und Mg-silikate | 0,24 |
| CMC | 0,06 |
| Hilfsstoffe (insgesamt) | 0,07 |
| | |
| Na-perborat-monohydrat | 0,71 g/l |
| | |
| Aktivator | 0,24 g/l |

Flottenverhältnis: 1 : 20
Waschflotte: 200 ml
Waschzeit: 15 Min. (Laborwaschgerät)
Wasserhärte: 14 °d
Spülen: 3 x 30 s

Die aus den Testanschmutzungen ermittelte Bleichwirkung (arithmetisches Mittel), angegeben als % Remissionszunahme (Bestimmung bei 460 nm; Datacolor Elrepho 2000; Xenonlampe, UV-Sperrfilter 420 nm; BaSO₄ als Standard (100 %)), folgt aus der Tabelle:

| Aktivator % | Remissionszunahme |
|---|---|
| (1) | 12,9 |
| (2) | 14,1 |
| (VB) | 13,7 |
| (TAED) | 14,4 |

Der erfindungsgemäße Aktivator (2) erreicht im Mittel etwa die Bleichleistung des TAED.

### Beispiel 10

### Bleichleistung des Aktivators (2) im Vergleich zu 1,3-Diacetylhydantoin (VB) und TAED bei 30 °C

Gewebe mit Testanschmutzungen sowie Meßmethode der Remissionszunahme wie Beispiel 9.

Die Prüfung erfolgt im Launderometer (Typ Atlas) unter Verwendung eines bleichmittel- und aktivatorfreien handelsüblichen Waschmittels aus dem US-Markt (TIDE-Ultra®).
- Waschflotte:: 200 ml
- Wasserhärte:: 5 °d
- Temperatur:: 30 °C
- Spülen:: Leitungswasser 3*30 s
- Flottenverhältnis:: 1 : 20 Ca:Mg = 2,7 : 1
- Waschzeit:: 15 Min.

Einsatzmengen (g) pro Waschflotte:

| | |
|---|---|
| Waschmittel | 0,27 |
| Na-perborat-monohydrat | 0,015 |
| Aktivator | 0,015 |

Die Remissionszunahme in % (arithemetisches Mittel aus den sechs Testanschmutzungen) folgen aus der Tabelle:

| Aktivator | % Remissionszunahme |
|---|---|
| (2) | 9,2 |
| (VB) | 8,8 |
| (TAED) | 8,7 |

Der erfindungsgemäße Aktivator (1) zeigt unter diesen Waschbedingungen gegenüber den Vergleichsprodukten eine überlegene Bleichleistung.

## Patentansprüche

1. Hydantoinderivate der allgemeinen Formel (I) 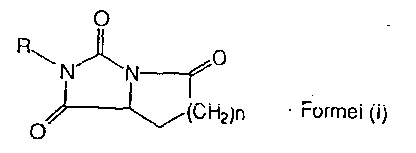 worin bedeuten
n die Zahl 1 oder 2 und
R Wasserstoff, (C₁- bis C₄)Alkyloxycarbonyl oder eine Acylgruppe der allgemeinen Formel R'-CO-, wobei
R' für einen linearen oder verzweigten gesättigten oder ungesättigten Kohlenwasserstoffrest mit 1 bis 11 C-Atomen, dessen längste Kette durch eine oder mehrere Etherbrücken unterbrochen sein kann und der zusätzlich ein oder zwei Substituenten aus der Reihe Carboxy, C₁- bis C₃-Alkoxy, -N((C₁-C₃)alkyl)₃⁺Cl⁻, Phenyl oder mit ein oder zwei Carboxy- oder
Sulfogruppen subtituiertes Phenyl aufweisen kann,
oder
für Phenyl oder ein- oder zweifach substituiertes Phenyl, wobei die Substituenten C₁- bis C₄-Alkyl, Carboxy, -SO₃H, -NO_{2,} -N((C₁-C₃)alkyl)₃⁺Cl⁻ oder -CN sein können,
oder
für einen Rest der Formel (II) 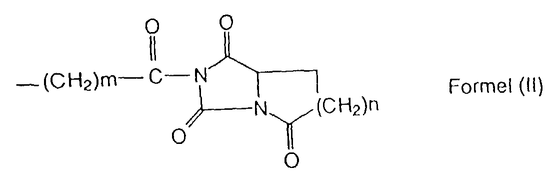 in welcher m eine ganze Zahl zwischen 2 und 10 und n die Zahl 1 oder 2 bedeuten, steht.

2. Hydantoinderivate nach Anspruch 1,
dadurch gekennzeichnet,
daß der Rest R Acetyl, Propionyl, Butyryl, Benzoyl oder o- oder p-Carboxybenzoyl ist.

3. Verwendung der Hydantoinderivate gemäß Anspruch 1 oder 2 als Aktivator für anorganische Persauerstoffverbindungen.

4. Verwendung nach Anspruch 3,
dadurch gekennzeichnet,
daß als Aktivator ein solcher gemäß Anspruch 2 und als anorganische Persauerstoffverbindung Wasserstoffperoxid, ein Perborat oder Percarbonat eingesetzt wird.

5. Bleich-, Wasch-, Reinigungs- oder Desinfektionsmittel, enthaltend eine anorganische Persauerstoffverbindung und einen Aktivator,
dadurch gekennzeichnet,
daß es als Aktivator ein Hydandoinderivat gemäß Anspruch 1 enthält.

6. Mittel nach Anspruch 5,
dadurch gekennzeichnet,
daß es pro Mol Aktivsauerstoff 0,05 bis 1 Mol Aktivator enthält.

7. Mittel nach Anspruch 5 oder 6,
dadurch gekennzeichnet,
daß es als anorganische Persauerstoffverbindung ein Perborat oder Percarbonat enthält.

8. Mittel nach einem der Ansprüche 5 bis 7,
dadurch gekennzeichnet,
daß es als Aktivator 2-Acetyl-, 2-Propionyl-, 2-Butytyl-, 2-Benzoyl-, 2-o- oder p-Carboxybenzoyldihydro-pyrrolo[1,2-c]imidazol-1,3,5-trion enthält.

## Claims

1. Hydantoin derivatives corresponding to the general formula (I) 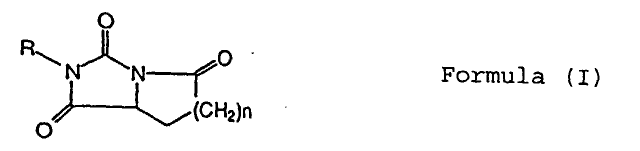 wherein
n is the number 1 or 2 and
R denotes hydrogen, (C₁ to C₄) alkyloxycarbonyl or an acyl group corresponding to the general formula R'-CO-, wherein
R' represents a linear or branched, saturated or unsaturated hydrocarbon group having 1 to 11 C atoms, whereof the longest chain can be interrupted by one or more ether bridges, which group can in addition have one or two substituents selected from amongst carboxy, C₁- to C₃-alkoxy, - N((C₁-C₃)alkyl)₃⁺Cl⁻, phenyl or phenyl substituted by one or two carboxy or sulpho groups, or
represents phenyl or mono- or disubstituted phenyl, wherein the substituents can be C₁- to C₄-alkyl, carboxy, -SO₃H, -NO₂, -N(C₁-C₃)alkyl)₃⁺Cl⁻ or -CN,
or
represents a group corresponding to formula (II) 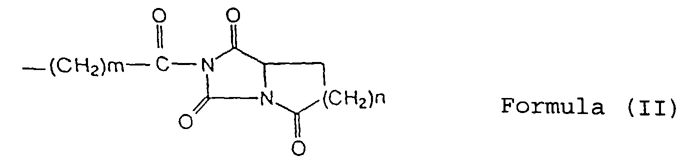 wherein m denotes an integer between 2 and 10 and n denotes the number 1 or 2.

2. Hydantoin derivatives according to claim 1, characterised in that the group R is acetyl, propionyl, butyryl, benzoyl or o- or p-carboxybenzoyl.

3. The use of the hydantoin derivatives according to claim 1 or 2 as activators for inorganic peroxy compounds.

4. The use according to claim 3, characterised in that the activator used is of a type according to claim 2 and the inorganic peroxy compound used is hydrogen peroxide, a perborate or percarbonate.

5. A bleaching agent, detergent, cleaning agent or disinfectant containing an inorganic peroxy compound and an activator, characterised in that it contains as activator a hydantoin derivative according to claim 1.

6. An agent according to claim 5, characterised in that it contains from 0.05 to 1 mol of activator per mol of active oxygen.

7. An agent according to claim 5 or 6, characterised in that it contains as inorganic peroxy compound a perborate or percarbonate.

8. An agent according to one of claims 5 to 7, characterised in that it contains as activator 2-acetyl-, 2-propionyl-, 2-butyryl-, 2-benzoyl-, 2-o- or 2-p-carboxybenzoyldihydropyrrolo[1,2-c]imidazol-1,3,5-trione.

## Revendications

1. Dérivés d'hydantoïne de formule générale (I). 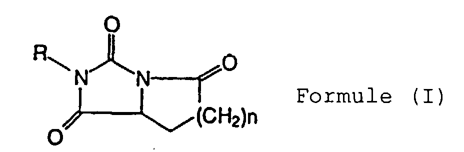 dans laquelle
n signifie le nombre 1 ou 2 et,
R signifie de l'hydrogène, un (alcoyle en C₁ à C₄) oxycarbonyle ou un groupe acyle de formule générale R'-CO dans laquelle :
R' représente un radical hydrocarboné linéaire ou ramifié, saturé ou non saturé, ayant de 1 à 11 atomes de carbone dont la chaîne la plus longue peut être interrompue par un ou plusieurs ponts éther, et qui peut posséder en supplément un ou deux substituants choisis dans la série du carboxy, de l'alcoxy en C₁ à C₃, du -N-(alcoyle en C₁ à C₃)₃ ⁺Cl⁻, du phényle ou phényl substitué par un ou deux groupes carboxy ou sulfo
ou représente un phényle ou un phényle substitué une fois ou deux fois, pour lequel les substituants peuvent être un alcoyle en C₁ à C₄, un carboxy, un -SO₃H, un -NO₂, un -N (alcoyle en C₁ à C₃)₃⁺Cl⁻ ou -CN,
ou représente un reste de formule (II) 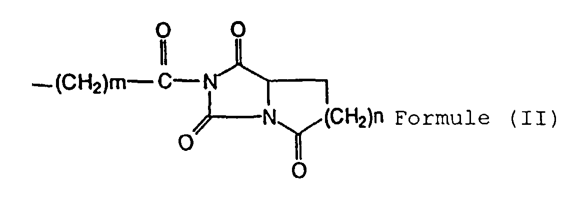 dans laquelle m signifie un nombre entier entre 2 et 10. et n signifie un nombre 1 ou 2.

2. Dérivés d'hydantoïne selon la revendication 1,
caractérisés en ce que
le radical R est un acétyle, un propionyle, un butyryle, un benzoyle ou un o- ou p- carboxybenzoyle.

3. Utilisation des dérivés d'hydantoïne selon la revendication 1 ou la revendication 2,
comme activateur pour des composés peroxydiques minéraux.

4. Utilisation selon la revendication 3,
caractérisée en ce que
comme activateur on met en oeuvre celui selon la revendication 2 et comme composé peroxydique minéral le peroxyde d'hydrogène, un perborate ou un percarbonate.

5. Produit de blanchiment, de lavage, de nettoyage ou de désinfection renfermant un composé peroxydique minéral et un activateur,
caractérisé en ce qu'
il renferme comme activateur un dérivé d'hydantoïne selon la revendication 1.

6. Produit selon la revendication 5,
caractérisé en ce qu'
il renferme par molécule d'oxygène actif, de 0,05 à 1 mol d'activateur.

7. Produit selon la revendication 5 ou la revendication 6,
caractérisé en ce qu'
il renferme comme composé peroxydique minéral, un perborate ou un percarbonate.

8. Produit selon l'une des revendications 5 à 7,
caractérisé en ce qu'
il renferme comme activateur la 2-acétyl, la 2-propionyl, la 2-butyryl-, la 2-benzoyl-, la 2-o-carboxy- ou p-carboxybenzoyl-dihydro-pyrrolo[1,2-c]imidazole-1,3,5-trione.
